# EUROPÄISCHE PATENTSCHRIFT

(11) **EP 1 532 095 B1**
(45) Veröffentlichungstag und Bekanntmachung des Hinweises auf die Patenterteilung: **27.01.2010**
(21) Anmeldenummer: 03794848.6
(22) Anmeldetag: 07.08.2003
(51) Int. Cl.: C07C 45/50

(54) **VERFAHREN ZUR HERSTELLUNG VON ALDEHYDEN DURCH HYDROFORMYLIERUNG VON OLEFINISCH UNGESÄTTIGTEN VERBINDUNGEN, KATALYSIERT DURCH UNMODIFIZIERTE METALLKOMPLEXE IN GEGENWART VON CYCLISCHEN KOHLENSÄUREESTERN**
METHOD FOR PRODUCING ALDEHYDES BY MEANS OF HYDROFORMYLATION OF OLEFINICALLY UNSATURATED COMPOUNDS, SAID HYDROFORMYLATION BEING CATALYSED BY UNMODIFIED METAL COMPLEXES IN THE PRESENCE OF CYCLIC CARBONIC ACID ESTERS
PROCEDE DE PRODUCTION D'ALDEHYDES PAR HYDROFORMYLATION DE COMPOSES INSATURES PAR OLEFINE, CATALYSEE PAR DES COMPLEXES METALLIQUES NON MODIFIES, EN PRESENCE D'ESTERS D'ACIDE CARBONIQUE CYCLIQUES

(30) Priorität: 31.08.2002 DE 10240253; 18.06.2003 DE 10327435
(43) Veröffentlichungstag der Anmeldung: 25.05.2005
(73) Patentinhaber: Evonik Oxeno GmbH, 45772 Marl (DE)
(72) Erfinder: MÖLLER, Oliver, 45739 Oer-Erkenschwick (DE); WIESE, Klaus-Diether, 45721 Haltern am See (DE); HESS, Dieter, 45770 Marl (DE); BORGMANN, Cornelia, 45657 Recklinghausen (DE); KAIZIG, Alfred, 45772 Marl (DE); FRIDAG, Dirk, 45721 Haltern am See (DE)
(74) Vertreter: Hirsch, Hans-Ludwig
(86) Internationale Anmeldenummer: PCT/EP2003/008737
(87) Internationale Veröffentlichungsnummer: WO 2004/024661

(56) Entgegenhaltungen:
- WO-A-87/07261
- DE-A- 3 511 428
- US-A- 4 490 559

## Beschreibung

Die vorliegende Erfindung betrifft ein Verfahren zur Herstellung von Aldehyden durch Hydroformylierung von olefinisch ungesättigten Verbindungen, insbesondere Olefinen, katalysiert durch einen unmodifizierten Metallkatalysator eines Metalls der 8. bis 10. Gruppe des Periodensystems der Elemente, das in Gegenwart von cyclischen Kohlensäureestern als Lösemittel durchgeführt wird.

Die Reaktionen zwischen Olefinverbindungen, Kohlenmonoxid und Wasserstoff in Gegenwart eines Katalysators zu den um ein C-Atom reicheren Aldehyden sind als Hydroformylierung (Oxierung) bekannt. Als Katalysatoren in diesen Reaktionen werden häufig Verbindungen der Übergangsmetalle der 8. bis 10. Gruppe des Periodensystems der Elemente verwendet, insbesondere Verbindungen des Rhodiums und des Kobalts. Die Hydroformylierung mit Rhodiumverbindungen bietet im Vergleich zur Katalyse mit Kobaltverbindungen in der Regel den Vorteil höherer Chemo- und Regioselektivität und ist damit meistens auch wirtschaftlich attraktiver.

Bei der durch Rhodium katalysierten Hydroformylierung werden zumeist Komplexe eingesetzt, die aus Rhodium und Verbindungen der 15. Gruppe des Periodensystems der Elemente, bevorzugt aus trivalenten Phosphorverbindungen als Liganden bestehen (WO-A-87/07261). Häufig werden als Liganden beispielsweise Verbindungen aus den Klassen der Phosphine, Phosphite und Phosphonite eingesetzt. Eine Übersicht über Hydroformylierung von Olefinen findet sich in B. CORNILS, W. A. HERRMANN, "Applied Homogeneous Catalysis with Organometallic Compounds", Vol. 1&2, VCH, Weinheim, New York, 1996.

Terminale Olefine lassen sich leicht in Gegenwart von Phosphin-modifizierten Rhodium-Katalysatoren umsetzen. Innenständige und ganz besonders innenständig-hochverzweigte Olefine erfordern dagegen stark aktivierende Liganden, wie z. B. Phosphit-Liganden. Bei schwer oxierbaren Olefinen hat sich daneben auch das sogenannte "nackte" oder unmodifizierte Rhodium als gut geeignet erwiesen. Es handelt sich dabei um eine oder mehrere Metall-Spezies, die sich unter Hydroformylierungsbedingungen aus einem Metall-Salz in Abwesenheit von modifizierenden Liganden bilden. Im Sinne dieser Anmeldung sind als modifizierende Liganden Verbindungen zu verstehen, die eines oder mehrere Donoratome der 15. Gruppe des Periodensystems der Elemente enthalten. Als modifizierende Liganden sollen jedoch nicht Alkoxy-, Carbonyl-, Hydrido-, Alkyl-, Aryl-, Allyl-, Acyl- oder Alken-Liganden gelten, sowie die Gegenionen der zur Katalysatorbildung eingesetzten Metall-Salze, z. B. Halogenide, wie beispielsweise Fluorid, Chlorid, Bromid oder Iodid, Acetylacetonat, Carboxylate, wie beispielsweise Acetat, 2-Ethylhexanoat, Hexanoat, Octanoat oder Nonanoat.

Modifizierende Liganden im Sinne dieser Anmeldung sind Liganden, die Donoratome aus der 15. Gruppe des Periodensystems der Elemente als enthalten, wie Stickstoff, Phosphor, Arsen oder Antimon und insbesondere Phosphor. Die Liganden können ein oder mehrzähnig sein, bei chiralen Liganden kann sowohl das Racemat als auch ein Enantiomer oder Diastereomer eingesetzt werden. Als Phosphorliganden sind insbesondere Phosphine, Phosphinine, Phosphinane, Phosphinoxide, Phosphite, Phosphonite und Phosphinite zu nennen.

Beispiele für Phosphine sind Triphenylphosphin, Tris(p-tolyl)phosphin, Tris(m-tolyl)phosphin, Tris(o-tolyl)phosphin, Tris(p-methoxyphenyl)phosphin, Tris(p-fluorphenyl)phosphin, Tris(p-chlorphenyl)phosphin, Tris(p-dimethylaminophenyl)phosphin, Ethyldiphenylphosphin, Propyldiphenylphosphin, t-Butyldiphenylphosphin, n-Butyldiphenylphosphin, n-Hexyldiphenylphosphin, c-Hexyldiphenylphosphin, Dicyclohexylphenylphosphin, Tricyclohexylphosphin, Tricyclopentylphosphin, Triethylphosphin, Tri-(1-naphthyl)phosphin, Tri-2-furylphosphin, Tribenzylphosphin, Benzyldiphenylphosphin, Tri-n-butylphosphin, Tri-i-butylphosphin, Tri-t-butylphosphin, Bis(2-methoxyphenyl)phenylphosphin, Neomenthyldiphenylphosphin, die Alkali-, Erdalkali-, Ammonium- oder andere Salze sulfonierter Triphenylphosphine wie Tris(m-sulfonylphenyl)phosphin, (m-Sulfonylphenyl)diphenylphosphin; 1,2-Bis(dicyclohexylphosphino)ethan, Bis(dicyclohexylphosphino)methan, 1,2-Bis(diethylphosphino)ethan, 1,2-Bis(2,5-diethylphospholano)benzol [Et-DUPHOS], 1,2-Bis(2,5-diethylphospholano)ethan [Et-BPE], 1,2-Bis(dimethylphosphino)ethan, Bis(dimethylphosphino)methan, 1,2-Bis(2,5-dimethylphospholano)benzol [Me-DUPHOS], 1,2-Bis(2,5-dimethylphospholano)ethan [Me-BPE], 1,2-Bis(diphenylphosphino)benzol, 2,3-Bis(diphenylphosphino)bicyclo[2.2.1]hept-5-en [NORPHOS], 2,2'-Bis(diphenylphosphino)-1,1'-binaphthyl [BINAP], 2,2'-Bis(diphenylphosphino)-1,1'-bipenyl [BISBI], 2,3-Bis(diphenylphosphino)butan, 1,4-Bis(diphenylphosphino)-butan, 1,2-Bis(diphenylphosphino)ethan, Bis(2-diphenylphosphinoethyl)phenylphosphin, 1,1'-Bis(diphenylphosphino)ferrocen, Bis(diphenylphosphino)methan, 1,2-Bis(diphenylphosphino)propan, 2,2'-Bis(di-p-tolylphosphino)-1,1'-binaphthyl, O-Isopropyliden-2,3-dihydroxy-1,4-bis(diphenylphosphino)butan [DIOP], 2-(Diphenylphosphino)-2'-methoxy-1,1'-binaphthyl, 1-(2-Diphenylphosphino-1-naphthayl)isochinolin, 1,1,1 -Tris(diphenylphosphino)ethan, und/oder Tris(hydroxypropyl)phosphin.

Beispiele für Phosphinine sind u. a. 2,6-Dimethyl-4-phenylphosphinin, 2,6-Bis(2,4-dimethylphenyl)-4-phenylphosphinin sowie weitere in WO 00/55164 beschriebene Liganden. Beispiele für Phosphinane sind u. a. 2,6-Bis(2,4-dimethylphenyl)-1-octyl-4-phenylphosphinan, 1-Octyl-2,4,6-triphenylphosphinan sowie weitere in WO 02/00669 beschriebene Liganden.

Beispiele für Phosphite sind Trimethylphosphit, Triethylphosphit, Tri-n-propylphosphit, Tri-i-propylphosphit, Tri-n-butylphosphit, Tri-i-butylphosphit, Tri-t-butylphosphit, Tris(2-ethylhexyl)phosphit, Triphenylphosphit, Tris(2.4-di-t-butylphenyl)phosphit, Tris(2-t-butyl-4-methoxyphenyl)phosphit, Tris(2-t-butyl-4-methylphenyl)phosphit, Tris(p-kresyl)phosphit. Außerdem sterisch gehinderte Phosphitliganden, wie sie unter anderem in EP 155 508, US 4 668 651, US 4 748 261, US 4 769 498, US 4 774 361, US 4 835 299, US 4 885 401, US 5 059 710, US 5 113 022, US 5 179 055, US 5 260 491, US 5 264 616, US 5 288 918, US 5 360 938, EP 472 071, EP 518 241 und WO 97/20795 beschriebenen werden. Bei den sterisch gehinderten Phosophiten sind die jeweils 1 oder 2 Isopropyl- und/oder tert.-Butyl-substituierten, vorzugsweise in ortho-Position zur Phosphitestergruppierung, substituierten Triphenylphosphite zu nennen. Weitere Bisphosphit-Liganden sind u.a. in EP 1 099 677, EP 1 099 678, WO 02/00670, JP 10279587, EP 472017, WO 01/21627, WO 97/40001, WO 97/40002, US 4769498, EP 213639 und EP 214622 genannt.

Beispiele für Phosphonite sind Methyldiethoxyphosphin, Phenyldimethoxyphosphin, Phenyldiphenoxyphosphin, 6-Phenoxy-6H-dibenz[c,e][1,2]oxaphosphorin und dessen Derivate, in denen die Wasserstoffatome ganz oder teilweise durch Alkyl-, Arylreste oder Halogenatome ersetzt sind und Liganden die in WO 98/43935, JP 09-268152 und DE 198 10 794 und in den deutschen Patentanmeldungen DE 199 54 721 und DE 199 54 510 beschrieben werden.

Gängige Phosphinitliganden sind unter anderem in US 5 710 344, WO 95 06627, US 5 360 938, JP 07082281 beschrieben. Beispiele hierfür sind Diphenyl(phenoxy)phosphin und dessen Derivate, in denen die Wasserstoffatome ganz oder teilweise durch Alkyl-, Arylreste oder Halogenatome ersetzt sind, Diphenyl(methoxy)phosphin, Diphenyl(ethoxy)phosphin usw.

In der technischen Ausführung der Hydroformylierung erfolgt die Trennung von Reaktionsprodukt, nicht umgesetztem Edukt und Katalysator meist durch Destillation. Die Hydroformylierung wird daher in Gegenwart eines hochsiedenden Lösemittels durchgeführt, so dass bei der destillativen Aufarbeitung u.a. eine hochsiedende katalysatorhaltige Fraktion erhalten wird, die in den Prozess zurückgeführt werden kann. In vielen technischen, kontinuierlichen Hydroformylierungsverfahren, bei denen Rhodiumkatalysatoren eingesetzt werden, kommen als Lösemittel die Hochsiedergemische, die als Nebenprodukt bei der Hydroformylierung entstehen, zum Einsatz, wie beispielsweise in DE 2 062 703, DE 2 715 685, DE 2 802 922, EP 017183 beschrieben ist.

Zusätzlich zu den Hochsiedem können inerte organische Flüssigkeiten (DE 3 126 265) sowie Reaktionsprodukte (Aldehyde, Alkohole), aliphatische und aromatische Kohlenwasserstoffe, Ester, Ether und Wasser (DE 4 419 898) als Lösemittel eingesetzt werden. In GB 1 197 902 werden zu diesem Zweck gesättigte Kohlenwasserstoffe, Aromaten, Alkohole und n-paraffine verwendet.

Die Durchführung der Hydroformylierung unter Zusatz eines oder mehrerer polarer organischer Stoffe werden z. B. in WO 01/68248, WO 01/68249, WO 01/68252 offengelegt. Dabei werden unter polaren Stoffen Substanzen aus folgenden Verbindungsklassen verstanden: Nitrile, cyclische Acetale, Alkohole, Pyrrolidone, Lactone, Formamide, Sulfoxide und Wasser.

Bei der Hydroformylierung längerkettiger Olefine (C ≥ 6) erfordert die destillative Abtrennung des Katalysators vom Reaktionsprodukt und ggf. der nicht umgesetzten Edukte hohe Temperaturen und niedrige Drücke. Dabei findet eine zum Teil erhebliche Zersetzung des rhodiumhaltigen Katalysators statt, unabhängig davon, ob ein zusätzlicher Ligand eingesetzt wurde oder nicht. Der Katalysator geht dabei für den Prozess verloren, wodurch die Wirtschaftlichkeit des Prozesses drastisch beeinträchtigt wird.

Die unmodifizierten Rhodium-Katalysatoren erweisen sich als besonders instabil. Nach übereinstimmender Auffassung der Fachwelt ist der mononukleare Komplex HRh(CO)₃, bei Abwesenheit modifizierender Liganden, die in der Hydroformylierung aktive Spezies. Der Komplex HRh(CO)₃ ist nur bei Temperaturen unter 20°C und Hochdruck stabil (N.S. Imyanitov, Rhodium Express, (1995), 10/11, 3 - 64) und steht im Gleichgewicht mit einer binuklearen Spezies, die selbst nicht aktiv ist, aber als Reservoir für aktiven Katalysator dient (E. V. Slivinskii, Y. A. Rozovskii, G. A. Korneeva, V. I. Kurkin, Kinetics and Catalysis (1998), 39(6), 764 - 774) (A. R. El'man, V. I. Kurkin, E. V. Slivinskii, S. M. Loktev, Neftekhimiya (1990), 30(1), 46 -52). Ausgehend vom bimolekularen Rhodium-Carbonyl-Komplex bilden sich hydroformylierungsinaktive Cluster mit zunehmendem Molekulargewicht. Unter den Bedingungen einer intensiven Hydroformylierungsreaktion ist die Bildung der niedermolekularen Cluster reversibel. Es ist nachgewiesen worden, dass Cluster bis hin zum Rh₄(CO)₁₂ regeneriert werden können. Die Stabilisierung der aktiven Spezies unter Hydroformylierungsbedingungen konnte ebenfalls gezeigt werden (Yu. B. Kagan, Y. A. Rozovskii, E. V. Slivinskii, G. A. Korneeva, V. I. Kurkin, S. M. Loktev, Kinetika i Kataliz (1987), 28(6), 1508 - 1511). Höhermolekulare Cluster lassen sich hingegen unter Hydroformylierungsbedingungen nicht wieder in aktive Spezies überführen (Yu. B. Kagan, E. V. Slivinskii, V. I. Kurkin, G. A. Korneeva, R. A. Aranovich, N. N. Rzhevskaya, S. M. Loktev, Neftekhimiya (1985), 25(6), 791 - 797.). Die Entstehung von Clustem gilt allgemein als Ursache und erster Schritt für Bildung fester rhodiumhaltiger Ausfällungen. Sie tritt während der destillativen Aufarbeitung, teilweise aber auch bereits unter Reaktionbedingungen auf. rhodiumhaltige Ausfällungen scheiden sich an Behälter- und Rohrwandungen ab. Das führt zu erheblichen wirtschaftlich nachteiligen Katalysatorverlusten und macht in der technischen Anwendung regelmäßige Anlagenabstellungen und Reinigungsarbeiten notwendig. Rhodium-Ausfällungen müssen auf metallurgischem Wege aufwändig zurückgewonnen werden.

Wegen der Attraktivität des unmodifizierten Rhodiums als Hydroformylierungskatalysator auf der einen Seite und seiner Instabilität auf der anderen sind viele Verfahren zu seiner Kreislaufführung und/oder Rückgewinnung vorgeschlagen worden.

Es sind eine Reihe von Verfahren bekannt, in denen eine Entfernung der Rhodium-Spezies aus der Reaktionsmischung durch feste Adsorbentien vorgenommen wird. So werden z. B. in der Schrift DE 19 54 315 als Adsorbens schwach bis stark basische Ionentauscherharze auf Polystyrolbasis vorgeschlagen. Gemäss DE 20 45 416 kann eine Regenerierung beladener Ionentauscherharze durch Behandlung mit Gemischen aus niederen Alkoholen, aliphatischen Aminen und Wasser in Gegenwart von Sauerstoff durchgeführt werden. Das im Eluat enthaltenen Rhodium wird durch Einengen und Behandeln mit Salzsäure ins Rhodiumchlorid-Hydrat überführt, das erneut als Katalysatorvorstufe eingesetzt werden kann. Die Schriften WO 02/20451 und US 5 208 194 beanspruchen die Rückgewinnung von Rhodium aus beladenen Ionentauschern durch Verbrennung derselben und Isolierung des Rhodiums als Oxid aus der anfallenden Asche. In US 4 388 279 werden als Adsorbentien Salze der Metalle der 1. und 2. Gruppe des Periodensystems der Elemente, zeolithische Molekularsiebe und Ionentauscherharze vorgeschlagen. WO 01/72679 beansprucht ein Verfahren zur Adsorption von Rhodium an Aktivkohle, Polykieselsäuren und Aluminumoxiden bei erhöhter Temperatur in Gegenwart von Wasserstoff. Die Patentschrift EP 0 355 837 beschreibt ein Verfahren zur Adsorption von Rhodium an basischen Ionentauscherharzen, die mit ionisch gebundenen Organophosphor-Liganden modifiziert sind. Die Regenerierung des Harzes erfolgt durch Eluierung mittels Organophosphor-Liganden-haltigen Lösung. Die Schrift WO 97/03938 beansprucht ein Verfahren zur Adsorption aktiver Rhodium-Spezies sowie von Verunreinigungen an sauren lonentauscherharzen. Die Regenerierung erfolgt im ersten Schritt durch Eluierung der Verunreinigungen mit einem neutralen Lösungsmittel, anschließend durch Eluierung der aktiven Rhodium-Spezies mit einem sauren Lösungsmittel. Der so zurückgewonnene Katalysator wird, ggf. nach Rehydrierung erneut in der Hydroformylierung eingesetzt.

Gemeinsamer Nachteil der adsorptiven Verfahren zur Rhodium-Rückgewinnung ist die nicht befriedigend gelöste Aufgabe der Wiederfreisetzung der aktiven Spezies. Dem Fachmann ist bekannt, dass die dazu vorgeschlagenen Lösemittel oder Lösemittelgemische in der Hydroformylierung nicht inert sind, sondern zu Nebenreaktionen führen. Saure Lösemittel induzieren beispielsweise die hochexotherme und schwer kontrollierbare Aldolisierung der Aldehyde. Alkohole und Amine gehen Kondensationsreaktionen mit Aldehyden ein und vermindern so die Produktausbeute. Es ist daher zwingend notwendig, vor der Rückführung des Katalysators die genannten Lösemittel oder Lösemittelgemische zu entfernen. Dieses Konzept wird dadurch technisch äußerst aufwändig und teuer. Eine gewisse industrielle Bedeutung hat hingegen die Adsorption an Ionentauschern mit anschließender Veraschung und metallurgischer Rhodium-Rückgewinnung erlangt. Dieses Verfahren ist technisch einfach, aber dennoch verbesserungswürdig: Es wird teurer basischer Ionentauscher als Verbrauchsmaterial eingesetzt und die Veraschung mit anschließender metallurgischer Aufarbeitung der Metalloxide ist mit weiteren äußerst aufwändigen Prozessschritten verbunden.

Darüber hinaus sind eine Reihe von Verfahren bekannt, in denen Rhodium aus dem Reaktoraustrag mit Lösungen unterschiedlicher Komplexbildner extrahiert und nach erneuter Freisetzung in den Hydroformylierungsreaktor zurückgeführt wird. So ist beispielsweise die Rhodium-katalysierte Hydroformylierung in Gegenwart protonierbarer stickstoffhaltiger Liganden, Extraktion des Rhodium-Komplexes mit wässriger Säure, Deprotonierung und Rückführung des Rhodiums in den Prozess aus DE 196 03 201 bekannt. In DE 4 230 871 wird die wässrige Lösung direkt in die Reaktion zurückgeführt. In EP 0 538 732 wird die Extraktion des Reaktoraustrags mit wässriger Phosphin-Lösung unter Synthesegas-Druck beansprucht. WO 97/03938 beansprucht als Komplexbildner wasserlösliche Polymere wie Polyacrylsäuren, Maleinsäure-Copolymeren sowie phosphono-methylierte Polyvinylamine, Polyethylenimine und Polyacrylamide. EP 0 588 225 beansprucht als Komplexbildner Pyridine, Chinoline, 2,2'-Bipyridine, 1,10-Phenanthroline, 2,2'-Bichinoline, 2,2',6',2"-Terpyridine und Porphyrine, ggf. in sulfonierter und/oder carboxylierter Form. Die bei der wässrigen Extraktion notwendigen Komplexbildner sind oft jedoch teuer und schwer zugänglich. Zudem erfordern diese Verfahren mit zwei zusätzlichen Schritten (Extraktion und Katalysator-Freisetzung) einen erhöhten technischen Aufwand.

Weiterhin sind Verfahren bekannt, in denen Rhodium-Ausfällungen bei der klassischen destillativen Aufarbeitung des Reaktoraustrags durch Zusatz Phosphor(III)-haltiger Liganden verhindert werden sollen (DE 33 38 340, US 4,400,547). Die Regenierung bzw. Wiederfreisetzung der hydroformylierungsaktiven Rhodium-Spezies erfolgt durch Oxidation der Phosphor(III)-Liganden. Nachteil dieses Verfahrens ist der kontinuierliche Stabilisator-Verbrauch. Die entstehenden Phosphor(V)-Verbindungen müssen ständig ausgeschleust werden, um eine Anreicherung im Reaktorsystem zu verhindern. Prinzipbedingt wird dabei auch ein Teil des Rhodiums in aktiver Form mit ausgetragen. Auch dieser Prozess ist deshalb technisch wie wirtschaftlich verbesserungswürdig.

In WO 82/03856 wird die Destillation des Reaktoraustrags der Hydroformylierung in Anwesenheit von Sauerstoff beansprucht. Unter der Einwirkung von Sauerstoff wird ein Teil der in der Hydroformylierung gebildeten Aldehyde zu den entsprechenden Carbonsäuren oxidiert, die mit den Rhodium-Spezies unter Bildung löslicher Rhodium-Carboxylate reagieren. Die Rhodium-Carboxylate können in den Prozess zurückgeführt werden. Nachteil dieses Verfahrens ist eine verminderte Ausbeute an Wertprodukt.

In der noch nicht veröffentlichten Anmeldung DE 102 40 253 wird die Durchführung der Hydroformylierung in Gegenwart von durch Phosphorliganden modifizierten Katalysatoren auf Basis von Metallen der 8. bis 10. Gruppe des Periodensystems der Elemente beschrieben, wobei als Lösemittel cyclische Kohlensäureester eingesetzt werden. Der Einsatz von unmodifizierten Metallkomplexen der Metalle der 8. bis 10. Gruppe des Periodensystems wird nicht beschrieben.

In JP 10-226662 wird ein Verfahren zur Hydroformylierung von olefinischen Verbindungen beschrieben, bei dem ein Rhodium-Katalysator mit einem Natriumsalz sulfonierter Triphenylphosphine als Cokatalysator, also ein modifizierter Katalysator eingesetzt wird. Die Reaktion wird in Gegenwart einer polaren Komponente sowie einer Carbonsäure durchgeführt. Als polare Komponenten kann z. B. auch Ethylencarbonat eingesetzt werden. Die polare Komponente kann gemeinsam mit der Säure und dem Katalysator in die Hydroformylierungsreaktion zurückgeführt werden. Das Verfahren ist allerdings nur zur Hydroformylierung der vergleichsweise reaktiven terminalen Olefine anwendbar. Bei innenständigen und insbesondere innenständig-hochverzweigten Olefinen ist die Aktivität des Katalysators für eine industrielle Anwendung bei weitem nicht ausreichend.

Die bisher bekannten Verfahren zur Kreislaufführung bzw. Rückgewinnung von Rhodium aus Prozessen, die unmodifiziertes Rhodium als Hydroformylierungskatalysator nutzen, sind sowohl technisch wie wirtschaftlich verbesserungswürdig.

Dem Stand der Technik entsprechend existiert kein technisch wie wirtschaftlich zufriedenstellendes Verfahren zur Hydroformylierung schwer oxierbarer Olefine katalysiert durch unmodifiziertes Rhodium. Es bestand somit die Aufgabe ein in dieser Hinsicht erheblich verbessertes Verfahren bereitzustellen, insbesondere ein Verfahren, bei dem die Katalysator-Rückgewinnung einfach erfolgen kann, das sich durch eine deutlich verminderte Katalysator-Desaktivierung auszeichnet und dadurch Verluste an Katalysator weitestgehend verhindert werden können.

Es wurde nun überraschend gefunden, dass bei der Hydroformylierung olefinisch ungesättigter Verbindungen Selektivität und Aktivität erhöht sowie die Aufarbeitung des Reaktionsgemischs erleichtert werden kann und die Katalysatorstabilität erheblich erhöht werden kann, wenn die durch unmodifiziertes Rhodium katalysierte Hydroformylierung in Gegenwart von cyclischen Kohlensäureestern als Lösemittel durchgeführt wird.

Gegenstand der vorliegenden Erfindung ist demnach ein Verfahren zur katalytischen Hydroformylierung von olefinisch ungesättigten Verbindungen mit 3 bis 24 Kohlenstoffatomen, wobei als Katalysator ein zumindest ein Metall der 8. bis 10. Gruppe des Periodensystems der Elemente, aufweisender unmodifizierter Katalysator eingesetzt wird, welches dadurch gekennzeichnet ist, dass die Hydroformylierung in Gegenwart mindestens eines cyclischen Kohlensäureesters der Formel I 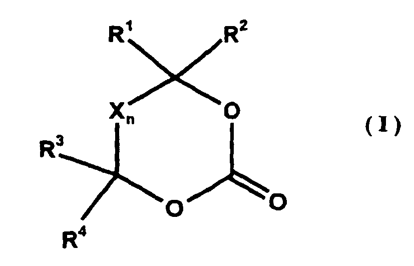 mit
R¹, R², R³, R⁴: jeweils gleich oder verschieden: H, substituierte oder unsubstituierte, aliphatische, alicyclische, aromatische, aliphatisch-alicyclische, aliphatisch-aromatische, alicyclisch-aromatische Kohlenwasserstoffreste mit 1 bis 27 C-Atomen. n: 0 - 5
X: zweiwertiger substituierter oder unsubstituierter, aliphatischer, alicyclischer, aromatischer, aliphatisch-alicyclischer, aliphatisch-aromatischer Kohlenwasserstoffrest mit 1 bis 27 C-Atomen,
durchgeführt wird, wobei der Anteil des Kohlensäureesters zumindest 1 Gew.-% des Reaktionsgemisches beträgt.

Durch den erfindungsgemäßen Einsatz von Kohlensäureestern als Lösemittel wird erreicht, dass die Hydroformylierung in Gegenwart von unmodifizierten Katalysator, insbesondere Rhodium-Katalysator durchgeführt werden kann, und der unmodifizierte Katalysator wiederverwendet werden kann.

Die bereits genannten, üblicherweise in der Rhodium-katalysierten Hydroformylierung eingesetzten modifizierten Liganden haben eine begrenzte thermische Stabilität, die die Reaktionstemperatur in der Regel auf 120 bis 130 °C beschränkt. Bei der Umsetzung schwer oxierbarer ethylenisch ungesättigter Verbindungen, wie z. B. innenständigen und insbesondere innenständig-hochverzweigten Olefinen, zeigen die ligandmodifizierten Rhodium-Katalysatoren bei Reaktionstemperaturen, die durch die thermische Stabilität der Liganden begrenzt sind und den üblichen Reaktionsdrücken von 1 bis 270 bar eine technisch unzureichende Aktivität.

Unmodifiziertes Rhodium weist dagegen bei der Umsetzung von schwer oxierbaren ethylenisch ungesättigten Verbindungen eine deutlich höhere Aktivität auf. Nachteilig ist jedoch die geringe thermische Stabilität (N.S. Imyanitov, Rhodium Express, (1995), 10/11, 3 - 64). Beispiele für schwer oxierbare ethylenisch ungesättigte Verbindungen sind innenständige und insbesondere innenständig-hochverzweigte Olefine, die in den Isomerengemischen enthalten sind, die durch Di- und Oligomerisierung von Propen und n-Buten erhalten werden, wie beispielsweise Tripropen, Tetrapropen, Dibuten, Tributen, Tetrabuten und Pentabuten.

Das erfindungsgemäße Verfahren hat insbesondere den Vorteil, dass der Katalysator eine erhöhte Langzeitstabilität gegenüber Katalysatoren aufweist, die in herkömmlichen Lösemitteln eingesetzt werden. Durch das verwendete Lösemittel ist außerdem die Abtrennung des Katalysators aus dem Reaktionsgemisch einfach möglich, da der Katalysator unabhängig von der Art der Aufarbeitung (destillativ oder über Phasentrennung) in der Phase vorliegt, in der auch der als Lösemittel verwendete cyclische Kohlensäureester vorhanden ist. Dieses Gemisch kann direkt wieder als Katalysatorlösung in den Hydroformylierungsreaktor gefahren werden. Die Auftrennung des Reaktionsaustrags durch Phasentrennung in eine Produkt und nichtumgesetztes Edukt enthaltende Fraktion und eine den Katalysator enthaltende Fraktion ist wesentlich schonender für den Katalysator als eine destillative Aufarbeitung. Eine thermische Belastung des Katalysators unter vermindertem Druck entfällt, weshalb die Entstehung von nichtaktiven Metallkatalysatorspezies und metallhaltigen Niederschlägen vermieden wird. Überraschenderweise wird auch bei der destillativen Auftrennung eine Desaktivierung durch Bildung der nichtaktiver Metallkatalysatorspezies und metallhaltiger Niederschläge weitestgehend verhindert.

Durch das erfindungsgemäße Verfahren ist es nun möglich, die Hydroformylierung von innenständig höchverzeigten Olefinen bei Temperaturen von bis zu 220 °C und unter Verwendung von Katalysatoren mit besonders hoher Aktivität durchzuführen. Auf diese Weise ist es möglich, den Umsatz und die Selektivität der Hydroformylierung insbesondere von innenständig hochverzweigten Olefinen zu steigern.

Das erfindungsgemäße Verfahren wird nachfolgend beispielhaft beschrieben, ohne dass die Erfindung auf diese beispielhaften Ausführungsformen beschränkt sein soll. Dem Fachmann ergeben sich weitere Varianten, die ebenfalls Gegenstand der vorliegenden Erfindung, deren Anwendungsbreite sich aus der Beschreibung und den Patentansprüchen ergibt, sind.

Das erfindungsgemäße Verfahren zur katalytischen Hydroformylierung von olefinisch ungesättigten Verbindungen mit 3 bis 24 Kohlenstoffatomen, insbesondere Olefinen, wobei als Katalysator ein zumindest ein Metall der 8. bis 10. Gruppe des Periodensystems der Elemente, aufweisender unmodifizierter Katalysator eingesetzt wird, zeichnet sich dadurch aus, dass die Hydroformylierung in Gegenwart mindestens eines cyclischen Kohlensäureesters der Formel I 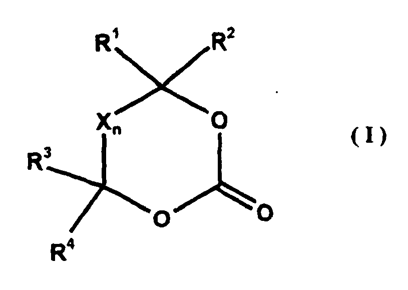 mit
- R¹, R², R³, R⁴_{:}: jeweils gleich oder verschieden: H, substituierte oder unsubstituierte, aliphatische, alicyclische, aromatische, aliphatisch-alicyclische, aliphatisch-aromatische, alicyclisch-aromatische Kohlenwasserstoffreste mit 1 bis 27 C-Atomen.
- n:: 0 - 5
- X:: zweiwertiger substituierter oder unsubstituierter, aliphatischer, alicyclischer, aromatischer, aliphatisch-alicyclischer, aliphatischaromatischer Kohlenwasserstoffrest mit 1 bis 27 C-Atomen,
durchgeführt wird, wobei der Anteil des Kohlensäureesters zumindest 1 Gew.-% des Reaktionsgemisches beträgt.

Die Substituenten R¹ bis R⁴ und X können gleich oder verschieden und durch O, N, NH-, N-alkyl oder N-dialkylreste substituiert sein. Weiterhin können diese Reste funktionelle Gruppen wie beispielsweise Halogene (Fluor, Chlor, Brom, Iod), -OH, -OR, -C(O)alkyl, -CN oder -C(O)Oalkyl tragen. Darüber hinaus können in diesen Resten C, CH- oder CH₂-Reste durch O , N, NH-, N-alkyl oder N-dialkylreste ersetzt sein, wenn sie mindestens drei C-Atome vom O-Atom der Estergruppe entfernt sind. Die Alkylgruppen können wiederum 1 bis 27 Kohlenstoffatome beinhalten.

Bevorzugt werden im erfindungsgemäßen Verfahren als cyclischer Kohlensäureester Ethylencarbonat, Propylencarbonat, Butylencarbonat oder deren Gemische, wie beispielsweise ein Gemisch (50 : 50 Gew.%) aus Ethylencarbonat und Propylencarbonat eingesetzt.

Im erfindungsgemäßen Verfahren beträgt der Anteil der cyclischen Kohlensäureester zwischen 1 und 98 Gew.-%, bevorzugt zwischen 5 und 70 Gew.-%, jedoch besonders bevorzugt zwischen 5 und 50 Gew.-% der Reaktionsmasse.

Es ist möglich, neben den cyclischen Kohlensäureestern weitere Lösemittel einzusetzen. In besonderen Verfahrensvarianten wird daher die erfindungsgemäße Hydroformylierungsreaktion in Gegenwart mindestens eines mit dem cyclischen Kohlensäureester I nicht mischbaren, unpolaren Lösemittels durchgeführt. Kohlensäureester der Formel I besitzen eine Dielektrizitätskonstante von über 30. Die im erfindungsgemäßen Verfahren eingesetzten, mit dem cyclischen Kohlensäureester I nicht mischbaren, unpolaren Lösemittel weisen Dₖ-Werte von kleiner 20, bevorzugt von 1,1 bis 10, besonders bevorzugt von 1,1 bis 5 auf. Durch die Verwendung von zusätzlichem, insbesondere unpolarem Lösemittel kann z. B. eingestellt werden, ob das Reaktionsgemisch und insbesondere der Reaktoraustrag, einphasig oder zweiphasig vorliegt. Somit kann gegebenenfalls eine in der Aufarbeitung des Reaktoraustrages vorgesehene Phasentrennung vereinfacht werden. Das Reaktionsprodukt der Hydroformylierung kann mit einem unpolaren und mit dem cyclischen Kohlensäureester I nicht mischbaren Lösemittel extrahiert werden, wobei das Lösemittel bereits während der Reaktion im Reaktionsgemisch vorhanden sein kann oder erst nach Beendigung der Reaktion hinzugefügt werden kann.

Als unpolare Lösemittel können substituierte oder unsubstituierte Kohlenwasserstoffe mit 10 bis 50 Kohlenstoffatomen wie z. B. die hochsiedenden Nebenprodukte der Hydroformylierungsreaktion, Texanol oder die Isomerengemische, die bei der Tetra- oder Pentamerisierung von Propen oder Buten mit nachfolgender Hydrierung erhalten werden, d. h. Tetrabutan, Pentabutan, Tetrapropan und/oder Pentapropan. Es ist ebenfalls möglich, Olefine mit 3 - 24 Kohlenstoffatomen, hier insbesondere das zur Hydroformylierung eingesetzte Olefin, als unpolares Lösemittel zu verwenden, indem die Hydroformylierungsreaktion nicht bis zum vollständigen Umsatz durchgeführt wird (z. B. nur bis zu 95 %, bevorzugt 90 %, besonders bevorzugt 80 %) und/oder weiteres Olefin während und/oder nach der Hydroformylierungsreaktion dem Reaktionsgemisch zugegeben wird.

Im erfindungsgemäßen Verfahren beträgt der Anteil der unpolaren Lösemittel zwischen 0 und 90 Gew.%, bevorzugt zwischen 5 und 50 Gew.%, besonders bevorzugt zwischen 5 und 30 Gew.% der Reaktionsmasse.

Zur Vermeidung von Nebenprodukten müssen die unpolaren Lösemittel, wenn es sich nicht um die eingesetzte olefinisch ungesättigte Verbindung handelt, unter den Reaktionsbedingungen der Hydroformylierungsreaktion weitestgehend inert sein.

Bei dem erfindungsgemäßen Verfahren kann das Reaktionsgemisch im Hydroformylierungsreaktor über den gesamten Umsatzbereich hinweg ein- oder zweiphasig sein. Es ist jedoch auch möglich, dass das Einsatzgemisch bei niedrigem Umsatz zunächst zweiphasig ist und im Verlauf der Reaktion bei höheren Umsätzen einphasig wird. Es ist möglich, dass ein einphasiges Eduktgemisch während der Durchführung des erfindungsgemäßen Verfahrens zu einem zweiphasigen Produktgemisch führt. Daneben kann das Phasenverhalten stark temperaturabhängig sein. Zum Beispiel kann ein bei Reaktionstemperatur einphasiges Reaktionsgemisch beim Abkühlen in zwei Phasen zerfallen. Ein bei Reaktionstemperatur zweiphasiges Reaktionsgemisch kann ebenso beim Abkühlen homogen werden.

Das erfindungsgemäße Verfahren kann mit verschiedenen katalytisch aktiven Metallen der 8. bis 10. Gruppe des Periodensystems der Elemente durchgeführt werden, bevorzugt jedoch mit Rhodium. Unter unmodifizierten, Metalle der 8. bis 10. Gruppe des Periodensystems der Elemente aufweisenden, Katalysatoren werden im Rahmen der vorliegenden Erfindung Katalysatoren verstanden, die keine modifizierende Liganden aufweisen. Im Sinne dieser Anmeldung sind als modifizierende Liganden Verbindungen zu verstehen, die eines oder mehrere Donoratome der 15. Gruppe des Periodensystems der Elemente enthalten. Als modifizierende Liganden sollen jedoch nicht Carbonyl-, Hydrido-, Alkoxy-, Alkyl-, Aryl-, Allyl-, Acyl- oder Alken-Liganden gelten, sowie die Gegenionen der zur Katalysatorbildung eingesetzten Metall-Salze, z. B. Halogenide, wie beispielsweise Fluorid, Chlorid, Bromid oder Iodid, Acetylacetonat, Carboxylate, wie beispielsweise Acetat, 2-Ethylhexanoat, Hexanoat, Octanoat oder Nonanoat. Ein besonders bevorzugter unmodifizierter Katalysator ist HRh(CO)₃.

Der aktive Katalysatorkomplex für die Hydroformylierungsreaktion wird dabei aus einem Salz oder einer Verbindung des Metalls (Katalysatorvorläufer) und Synthesegas gebildet.

Zweckmäßig geschieht dies in situ während der Hydroformylierung. Übliche Katalysatorvorläufer sind Rh(I)-, Rh(II)- und Rh(III)-Salze, beispielsweise Acetate, Octanoate, Nonanoate, Acetylacetonate oder Halogenide sowie die Rhodiumcarbonyle. Die Konzentration des Metalls im Reaktionsgemisch liegt vorzugsweise im Bereich von 1 ppm bis 1000 ppm, bevorzugt im Bereich von 5 ppm bis 300 ppm.

Die Edukte für eine Hydroformylierung gemäss dem Verfahren der Erfindung sind Verbindungen, die ethylenisch ungesättigte C-C-Doppelbindungen enthalten, insbesondere Olefine oder Gemische von Olefinen, insbesondere Monoolefine mit 3 bis 24, bevorzugt 4 bis 16, besonders bevorzugt 4 bis 12 Kohlenstoffatomen mit end- oder innenständigen C-C-Doppelbindungen, wie z. B. 1- oder 2-Penten, 2-Methylbuten-1, 2-Methylbuten-2, 3-Methylbuten-1, 1-, 2- oder 3-Hexen, das bei der Dimerisierung von Propen anfallende C₆-Olefingemisch (Dipropen), Heptene, 2- oder 3-Methyl-1-hexen, Octene, 2-Methylheptene, 3-Methylheptene, 5-Methylhepten-2, 6-Methylhepten-2, 2-Ethylhexen-1, das bei der Dimerisierung von n-Butenen anfallende Isomere C₈-Olefingemisch (Dibuten), das bei der Dimerisierung von iso-Buten anfallende C₈-Olefingemisch (Di-iso-Buten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung von Propen oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische, gegebenenfalls nach destillativer Trennung in Fraktionen mit gleicher oder ähnlicher Kettenlänge. Ebenfalls können Olefine oder Olefmgemische, die durch Fischer-Tropsch-Synthese, sowie Olefine, die durch Oligomerisierung von Ethen erhalten wurden oder Olefine, die über Methathesereaktionen zugänglich sind, eingesetzt werden. Bevorzugte Edukte sind C₄-, C₆-,C₈-, C₉-, C₁₂- oder C₁₆-Olefingemische. Das erfindungsgemäße Verfahren kann darüber hinaus zur Hydroformylierung polymerer olefinisch ungesättigter Verbindungen wie Polyisobuten oder 1,3-Butadien- oder Isobuten-Copolymeren eingesetzt werden. Die molare Masse der polymeren Olefine ist dabei unerheblich, das Olefin muss lediglich im Hydroformylierungsmedium ausreichend löslich sein. Die molare Masse der polymeren Olefine liegt bevorzugt unterhalb von 10000 g/mol, besonders bevorzugt unterhalb von 5000 g/mol.

Das Volumenverhältnis von Kohlenmonoxid zu Wasserstoff im Synthesegas liegt im Allgemeinen im Bereich von 2:1 bis 1:2, insbesondere bei einem Volumenverhältnis von 1:1. Das Synthesegas wird vorteilhaft im Überschuss, zum Beispiel bis zum Dreifachen der stöchiometrischen Menge, verwendet.

Die Hydroformylierungen werden in der Regel bei Drücken von 1 bis 350 bar durchgeführt, vorzugsweise bei Drücken von 15 bis 270 bar. Der angewendete Druck hängt von der Struktur der Einsatzolefine, dem eingesetzten Katalysator und dem gewünschten Effekt ab. So können beispielsweise α-Olefine unter Rhodium-Katalyse bei Drücken unter 100 bar mit hohen Raum-Zeit-Ausbeuten zu den entsprechenden Aldehyden umgesetzt werden. Bei Olefinen mit innenständigen Doppelbindungen, insbesondere bei verzweigten Olefinen, sind dagegen höhere Drücke zweckmäßig.

Die Reaktionstemperaturen des erfmdungsgemäßen Verfahrens betragen vorzugsweise von 20 bis 220 °C, bevorzugt von 100 °C bis 200 °C, vorzugsweise von 150 °C bis 190 °C, besonders bevorzugt von 160 bis 180 °C. Durch eine Reaktionstemperatur von über 150 °C kann insbesondere das Verhältnis von endständigen zu innenständigen Doppelbindungen verbessert werden, da bei den höheren Temperaturen durch beschleunigte Isomerisierung mehr endständige Doppelbindungen zur Verfügung gestellt werden und damit auch eine Hydroformylierung in der bevorzugten endständigen Position vermehrt stattfindet.

Das erfindungsgemäße Verfahren kann diskontinuierlich (batchweise) oder kontinuierlich durchgeführt werden. Bevorzugt wird jedoch eine kontinuierliche Betriebsweise. Als Reaktoren sind praktisch alle dem Fachmann bekannten Gas-Flüssig-Reaktoren geeignet, beispielsweise begaste Rührkessel oder Blasensäulen sowie Rohrreaktoren mit oder ohne Rückführung. Bevorzugt werden kaskadierte Blasensäulen und Rohrreaktoren mit statischen Mischelementen eingesetzt.

Der bei dem erfindungsgemäßen Verfahren erhaltene Reaktoraustrag weist ggf. nicht umgesetzte olefinisch ungesättigte Verbindung (Olefine), Reaktionsprodukte, Reaktionsnebenprodukte, mindestens einen cyclischen Kohlensäureester, ggf. ein unpolares Lösemittel und den Katalysator auf. Je nach Art und Massenanteil der als Einsatzstoff verwendeten olefinischen Verbindung(en), Art und Massenanteil des ggf. anwesenden unpolaren Lösemittels sowie Art und Massenanteil des cyclischen Kohlensäureesters kann der Reaktoraustrag ein- oder zweiphasig sein. Wie bereits oben erwähnt kann durch entsprechende Zugaben von cyclischen Kohlensäureestern oder unpolarem Lösemittel eine Phasentrennung erzielt oder verhindert werden.

Die Aufarbeitung des Reaktoraustrages kann im erfindungsgemäßen Verfahren, je nach dem Phasenverhalten des Reaktoraustrags, in zwei Varianten erfolgen. Bei einem zweiphasigen Reaktoraustrag wird bevorzugt eine Aufarbeitung über Phasentrennung gemäss **Variante A,** bei einem einphasigen Reaktoraustrag bevorzugt eine destillative Aufarbeitung nach **Variante B** angewendet.

Es kann vorteilhaft sein, wenn nach der Hydroformylierung der größte Teil des Synthesegases durch Druckentspannung entfernt wird, bevor die weitere Aufarbeitung des Reaktoraustrags gemäß Variante A oder B erfolgt.

### Variante A

In dieser Verfahrensvariante wird der zweiphasige Reaktoraustrag der Hydroformylierungsreaktion bevorzugt durch Trennung der Phasen in eine überwiegend den Katalysator und den oder die cyclischen Kohlensäureester enthaltende und eine überwiegend die Hydroformylierungsprodukte und nicht umgesetzte Olefine bzw. olefinisch ungesättigte Verbindungen enthaltende Fraktion getrennt.

Diese Verfahrensvariante bietet sich bei Verwendung eines optionalen weiteren unpolaren Lösungsmittels an. Das unpolare Lösungsmittel kann auch identisch mit dem Einsatzolefin sein, so dass entweder die Hydroformylierungsreaktion nicht bis zum vollständigen Umsatz durchgeführt wird (z. B. nur bis zu 95 %, bevorzugt 90 %, besonders bevorzugt 80 %) und/oder weiteres Olefin während und/oder nach der Hydroformylierungsreaktion dem Reaktionsgemisch zugegeben wird.

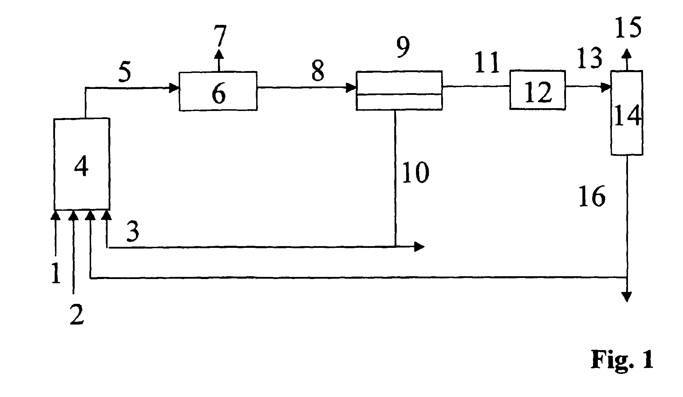

Die Variante A des erfindungsgemäßen Verfahrens wird durch Fig. 1 näher erläutert, ohne dass das Verfahren auf diese Ausführungsvariante beschränkt sein soll: Synthesegas (1), Olefine (2) und Hydroformylierungskatalysator gelöst in cyclischem Kohlensäureester oder einem Gemisch mehrerer cyclischer Kohlensäureester (3) werden im Hydroformylierungsreaktor (4) umgesetzt. Der Reaktoraustrag (5) kann optional in einem Entspannungsbehälter (6) vom überschüssigen Synthesegas (7) befreit werden. Der so erhaltene Stoffstrom (8) wird bevorzugt in einer Trennapparatur (9) in eine schwere Phase (10), die den größten Teil des cyclischen Kohlensäureesters und des Katalysators sowie hochsiedende Nebenprodukte enthält und eine leichte Phase (11), die die Hydroformylierungsprodukte, nicht umgesetztes Olefin und ggf. das unpolare Solvens enthalten, getrennt. Die Phasentrennung kann bei Temperaturen von 0 °C bis 130 °C, bevorzugt jedoch zwischen 10 °C und 60 °C vorgenommen werden. Die Phasentrennung kann z. B. in einem Settlerbehälter durchgeführt werden. Vorzugsweise wird die Phasentrennung in der Trennapparatur (9) unter Synthesegas bei einem Druck von 1 bis 350 bar, bevorzugt bei 15 bis 270 bar, besonders bevorzugt jedoch bei demselben Druck durchgeführt, der im Hydroformylierungsreaktor (4) angewendet wird. Der Trennapparatur (9) kann optional ein Wärmetauscher zum Abkühlen des Stoffstroms (5) vorgeschaltet sein (in Fig. 1 nicht gezeigt). In einer optionalen Trennstufe (12) können Katalysatorreste aus dem Strom (11) entfernt werden. Stoffstrom (11) oder (13) werden nun der Trennstufe (14) zugeführt. Hier werden die Reaktionsprodukte (Aldehyde und Alkohole) und nicht umgesetzte Olefine (15) abgetrennt und einer weiteren Aufarbeitung bzw. Hydrierung zugeführt. Das aus dem Stoffstrom (15) abgetrennte Olefin kann in denselben Reaktor oder in eine optionale zweite Reaktionsstufe geführt werden. Die ebenfalls abgetrennte Fraktion (16) enthält z. B. Reste des cyclischen Kohlensäureesters, der Reaktionsprodukte, der ggf. weiteren zugesetzten unpolaren Lösungsmittel und hochsiedende Nebenprodukte. Fraktion (16) kann in den Hydroformylierungsreaktor (4) verworfen oder zurückgeführt werden. Zweckmäßigerweise wird vor der Rückführung eine Aufarbeitung vorgenommen, bei der nicht erwünschte Nebenprodukte ausgeschleust werden. Die Katalysatorabtrennung in der Trennapparatur (9) kann als Extraktion durchgeführt werden, in dem mindestens ein Teil der Fraktion (16) und/oder mindestens ein Teil des Frischolefins (2) direkt dem Stoffstrom (8) zugeführt wird. Die Extraktion wird bevorzugt kontinuierlich durchgeführt, sie kann einstufig sein oder als mehrstufiger Prozess im Gegen-, Gleich- oder Kreuzstrom betrieben werden. katalysatorhaltige Ausschleuseströme, wie etwa aus dem Strom (10) oder der Trennstufe (12), können nach bekannten Verfahren auf das Katalysatormetall in wiederverwertbarer Form aufgearbeitet werden.

### Variante B

In dieser Verfahrensvariante wird der homogene Reaktoraustrag der Hydroformylierungsreaktion durch Destillation in eine leichtersiedende, überwiegend die Hydroformylierungsprodukte und ggf. nicht umgesetzte Olefine bzw. olefinisch ungesättigte Verbindungen enthaltende Fraktion und eine, überwiegend cyclischen Kohlensäureester und Katalysator aufweisende, höhersiedende Fraktion getrennt.

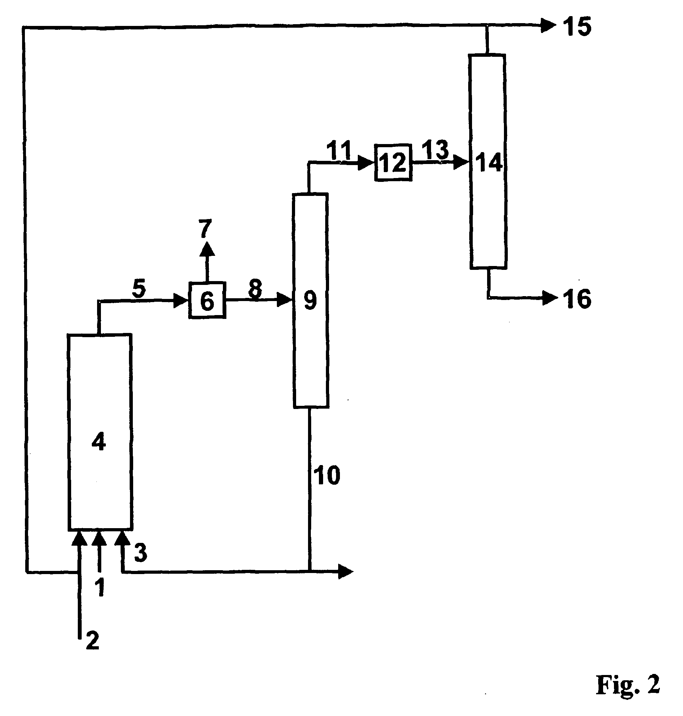

Die Variante B des erfindungsgemäßen Verfahrens wird durch Fig. 2 näher erläutert, ohne dass das Verfahren auf diese Ausführungsvariante beschränkt sein soll: Synthesegas (1), Olefine (2) und Hydroformylierungskatalysator, gelöst in cyclischem Kohlensäureester oder einem Gemisch mehrerer cyclischer Kohlensäureester (3), werden im Hydroformylierungsreaktor (4) umgesetzt. Der Reaktoraustrag (5) kann optional in einem Entspannungsbehälter (6) vom überschüssigen Synthesegas (7) befreit werden. Der so erhaltene Stoffstrom (8) wird in eine Trennapparatur (9) geführt und destillativ in eine schwersiedende Fraktion (10), die den größten Teil des cyclischen Kohlensäureesters und des Katalysators enthält und eine leichtsiedende Phase (11), die die Hydroformylierungsprodukte, nicht umgesetztes Olefin und ggf. unpolares Solvens enthält, getrennt. Die katalysatorhaltige Fraktion (10) wird in den Hydroformylierungsreaktor zurückgeführt. Vorher kann optional ein Aufarbeitungsschritt erfolgen, in dem hochsiedende Nebenprodukte und/oder Katalysatorabbauprodukte ausgeschleust werden (in Fig.2 nicht gezeigt). Fraktion (11) kann optional in einem Trennschritt (12) noch von Katalysatorresten befreit werden. Der Strom 13 wird dann der Destillationsstufe (14) zugeführt. Hier werden die Hydroformylierungsprodukte (Aldehyde und Alkohole) (16) destillativ vom nichtumgesetzten Olefin (15) getrennt. katalysatorhaltige Ausschleuseströme, wie etwa aus dem Strom (10) oder der Trennstufe (12), können nach Verfahren, die dem Fachmann z. B. aus WO 02/20451 oder US 5,208,194 bekannt sind, auf das Katalysatormetall in einer wiederverwertbaren Form aufgearbeitet werden. Die Hydroformylierungsprodukte können anschließend weiter aufgearbeitet werden.

Nichtumgesetztes Olefin (15) kann in denselben Hydroformylierungsreaktor oder in eine optionale zweite Reaktionsstufe geführt werden. In der technischen Ausführung der Trennapparaturen sind verschiedene Varianten anwendbar. Bevorzugt ist die Abtrennung über Fallfilm-, Kurzstrecken- oder Dünnschichtverdampfer oder Kombinationen aus diesen Apparaten. Der Vorteil einer solchen Kombination kann zum Beispiel darin liegen, in einem ersten Schritt noch gelöstes Synthesegas sowie den größten Teil der Produkte und der nichtumgesetzten Edukte von der katalystorhaltigen Alkylencarbonat-Lösung abzutrennen (zum Beispiel in einem Fallfilmverdampfer oder Flashverdampfer), um dann in einem zweiten Schritt (zum Beispiel in einer Kombination von zwei Kolonnen), die Abtrennung der restlichen Alkylencarbonate sowie die Trennung von Produkten und nichtumgesetzten Edukten vorzunehmen.

Die bei beiden Ausführungsvarianten A und B des erfindungsgemäßen Verfahrens erhaltenen, von Katalysator, überschüssigem Synthesegas und dem größten Teil des Lösemittels (also dem cyclischen Kohlensäureester bzw. einem Gemisch mehrerer) befreiten Reaktorausträge werden vorzugsweise weiter in Aldehyde (Alkohole), Olefine, Lösemittel und Nebenprodukte aufgetrennt. Dies kann, wie bekannt, z. B. durch Destillation geschehen. Aus dem Reaktionsaustrag oder den Hydroformylierungsprodukten abgetrenntes Olefin und/oder Lösemittel (Alkylencarbonat und/oder unpolares Lösemittel) können in die Hydroformylierungsreaktion rückgeführt werden.

Die genannten Varianten des erfindungsgemäßen Verfahrens beinhalten die Trennung des Reaktoraustrags und optional der Hydroformylierungsprodukte; dies kann beispielsweise durch Destillation erfolgen. Daneben ist allerdings auch ein Einsatz anderer Trennverfahren z. B. der Extraktion, wie u.a. in WO 01/68247, EP 0 922 691, WO 99/38832, US 5 648 554 und US 5 138 101 beschrieben oder Permeation, wie u.a. in DE 1953641, GB 1312076, NL 8700881, DE 3842819, WO 9419104, DE 19632600 und EP 1103303 beschrieben, möglich. Für die technische Ausführung der Trennung sind verschiedene Verfahrensweisen anwendbar. Bevorzugt ist die Abtrennung über Fallfilm-, Kurzstrecken- oder Dünnschichtverdampfer oder Kombinationen aus diesen Apparaten. Die extraktive Abtrennung wird vorteilhafterweise kontinuierlich durchgeführt. Sie kann als einstufiger Prozess ausgeführt sein oder als mehrstufiger Prozess im Gegenstrom oder Kreuzstrom betrieben werden.

In allen Verfahrensvarianten wird zweckmäßiger Weise die den Katalysator enthaltende Fraktion in die Hydroformylierungsreaktion rückgeführt. Dies ist selbstverständlich unabhängig von der Zusammensetzung der Fraktionen in der der Katalysator gelöst ist.

Wenn nicht die Aldehyde selbst, sondern die von ihnen abgeleiteten Alkohole Zielprodukte sind, kann der von Synthesegas und Katalysator und gegebenenfalls von Lösemittel befreite Reaktionsaustrag vor oder nach Olefinabtrennung hydriert werden und anschließend destillativ auf Reinalkohol aufgearbeitet werden.

Das erfindungsgemäße Verfahren kann ein- oder mehrstufig durchgeführt werden. Hierbei ist es möglich, nach einer ersten Hydroformylierungsreaktion eine zweite Hydroformylierungsstufe zu durchlaufen, die bei drastischeren Reaktionsbedingungen (zum Beispiel höhere Temperatur und/oder höherer Druck) auch die schwer hydroformylierbaren, innenständigen, insbesondere die innenständig-hochverzweigten Olefine zu den gewünschten Aldehyden umsetzt. Bevorzugt wird jedoch zunächst eine Trennung von nicht umgesetzten Olefinen und Hydroformylierungsprodukten (Aldehyden und Alkoholen) durchgeführt und die nicht umgesetzten Olefine in dieselbe Hydroformylierungsstufe zurückgeführt oder einer zweiten Hydroformylierungsstufe oder noch weiteren Hydroformylierungsstufen zugeführt. Dabei ist es auch möglich, in der zweiten Hydroformylierungsstufe ein völlig anderes Katalysatorsystem, d.h. ein anderes Katalysator-Metall oder ein Ligand-modifiziertes Katalysator-Metall, einzusetzen. Es kann darüber hinaus zweckmäßig sein, den nicht umgesetzten Olefinen darin eine höhere Konzentration an Katalysator zuzufügen, um auch schwerer hydroformylierbare Olefine zu den gewünschten Produkten umzusetzen. In allen Fällen ist es erforderlich, in den weiteren Hydroformylierungsstufen die cyclischen Kohlensäureester in den genannten Mengen zuzufügen.

Bei dem erfindungsgemäßen Verfahren können als olefinisch ungesättigte Verbindungen auch solche eingesetzt werden, die als nicht umgesetzte olefinisch ungesättigte Verbindungen aus dem Reaktoraustrag einer ersten Hydroformylierungsreaktion erhalten wurden. Dabei kann der gesamte Reaktionsaustrag oder aber nur ein Teil davon, insbesondere ein Teil, der den überwiegenden Teil der nicht umgesetzten olefinischen Verbindungen aus der ersten Stufe aufweist, eingesetzt werden. Es kann durchaus vorteilhaft sein, wenn bei dieser Verfahrensvariante die erste Hydroformylierungsreaktion in Gegenwart eines Ligand-modifizierten Katalysators durchgeführt wurde.

Die folgenden Beispiele sollen ausschließlich der Erläuterung der Erfindung dienen, nicht jedoch ihre Anwendungsbreite einschränken, die sich ausschließlich aus der Beschreibung und den Patentansprüchen ergibt.

### Beispiel 1 (Variante A)

In einem 21-Rührautoklav wurden unter Stickstoffatmosphäre 560 g Propylencarbonat, 560 g Tri-n-Buten und 0.0888 g bzw. 0.0225 g Rhodium(II)-nonanoat vorgelegt, das entspricht einer Rhodium-Konzentration von 5 ppm bzw. 20 ppm Rhodium bezogen auf die Masse des Reaktorinhalts. Der Autoklav wurde anschließend mit Synthesegas (CO/H₂ 1:1 molar) beaufschlagt und bis zur gewünschten Reaktionstemperatur aufgeheizt. Während des Aufheizens wurde der Reaktordruck kontrolliert. Die Reaktionstemperaturen betrugen von 130°C bis 180°C. Der Reaktionsdruck betrug 260 bar. Während der Reaktion wurde Synthesegas druckgeregelt nachgefahren. Nach 5 h wurde der Versuch abgebrochen und der Reaktor auf Umgebungstemperatur abgekühlt. Der Reaktoraustrag war stets zweiphasig und frei von Rhodium-Ausfällungen.

Die Zusammensetzung der in einem Phasentrennbehälter abgetrennten leichteren Kohlenwasserstoff-Phase wurde mittels Gaschromatographie ermittelt. Die Ergebnisse der Gaschromatographie sowie die Reaktionsbedingungen wie Temperatur und Rhodiumkonzentration sind in Tabelle 1 zusammengefasst.

**Tabelle 1: Hydroformylierung von Tri-n-Buten bei 260 bar und verschiedenen Temperaturen für 5 Stunden. Die angegebenen Anteile (in Massen-%) beziehen sich auf die Zusammensetzung der leichteren Kohlenwasserstoff-Phase, wobei gegebenenfalls vorhandener Kohlensäureester und Katalysator herausgerechnet wurden. In Versuch 6 wurde die bei der Aufarbeitung des Reaktoraustrages von Versuch 5 erhaltene Katalysatorlösung erneut eingesetzt.**

| Nr. | T / °C | c(Rh) / ppm | C13-Aldehyde / % | C13-Alkohole / % | C12-KWST / % | Hochsieder / % |
|---|---|---|---|---|---|---|
| 1 | 130 | 5 | 27 | 1 | 72 | 0 |
| 2 | 130 | 20 | 55 | 4 | 40 | 1 |
| 3 | 150 | 20 | 68 | 14 | 17 | 1 |
| 4 | 180 | 5 | 48 | 33 | 14 | 5 |
| 5 | 180 | 20 | 59 | 32 | 8 | 1 |
| 6 | 180 | 20 | 61 | 30 | 8 | 1 |

### Beispiel 2 (Variante B)

Analog zu Beispiel 1 wurde Di-n-Buten (560 g) hydroformyliert. Die Reaktorausträge der Versuche 7 bis 13 waren stets einphasig und frei von (Rhodium-)Ausfällungen. Im Unterschied zu Beispiel 1 wurde der Reaktoraustrag ohne Aufarbeitung gaschromatographisch analysiert. Die Ergebnisse der Gaschromatographie sowie die Reaktionsbedingungen wie Temperatur, Druck und Rhodiumkonzentration sind in Tabelle 2 zusammengefasst.

**Tabelle 2: Hydroformylierung von Di-n-Buten bei unterschiedlichen Drücken, Rhodiumkonzentrationen und Temperaturen. Die angegebenen Anteile (in Massen-%) beziehen sich auf die Zusammensetzung des Reaktoraustrags, wobei vorhandener Kohlensäureester und Katalysator herausgerechnet wurden.**

| Nr. | T / °C | p / bar | c(Rh) / ppm | C8-KWST / % | C9-Aldehyde / % | C9-Alkohole / % |
|---|---|---|---|---|---|---|
| 7 | 150 | 50 | 40 | 67.5 | 30.4 | 2.1 |
| 8 | 150 | 250 | 40 | 3,1 | 87.6 | 3.3 |
| 9 | 170 | 150 | 5 | 26.3 | 66.6 | 27.1 |
| 10 | 170 | 250 | 5 | 4.5 | 78.1 | 17.4 |
| 11 | 170 | 250 | 40 | 4.0 | 20.5 | 75.5 |
| 12 | 180 | 50 | 40 | 66.8 | 17.5 | 15.7 |
| 13 | 180 | 150 | 40 | 9.9 | 23.3 | 66.8 |

### Beispiel 3 (herkömmliche Vorgehensweise)

Wie in Beispiel 2 wurde Di-n-Buten hydroformyliert mit dem Unterschied, dass Pentabutan anstelle von Propylencarbonat als Lösemittel eingesetzt wurde. Der Reaktoraustrag aus Versuch 14 zeigte bereits deutliche schwarze (Rhodium-)Ausfällungen. Am Dünnschichtverdampfer wurden anschließend die Aldehyde und nichtumgesetzten Olefme von der katalysatorhaltigen Lösung abgetrennt, die in einer erneuten Hydroformylierung eingesetzt wurde (Versuch 15). Die Ergebnisse der Gaschromatographie sowie die Reaktionsbedingungen wie Temperatur, Druck und Rhodiumkonzentration sind in Tabelle 2 zusammengefasst.

**Tabelle 3: Hydroformylierung von Di-n-Buten bei 150 °C und 250 bar in Pentabutan. Die angegebenen Anteile (in Massen-%) beziehen sich auf die Zusammensetzung des Reaktoraustrags, wobei vorhandenes Pentabutan, Nebenprodukt und Katalysator herausgerechnet wurden. In Versuch 15 wurde die bei der destillativen Aufarbeitung von Versuch 14 erhaltene Katalysatorlösung erneut eingesetzt.**

| Nr. | T / °C | p / bar | c(Rh) / ppm | C8-KWST / % | C9-Aldehyde / % | C9-Alkohole / % |
|---|---|---|---|---|---|---|
| 14 | 150 | 250 | 40 | 75.4 | 23.4 | 1.2 |
| 15 | 150 | 250 | nicht bekannt | 91.5 | 7.8 | 0.7 |

Das Fehlen jeglicher Rhodium-Ausfällungen in den Versuchen 1 bis 13 deutet darauf hin, dass das als Lösemittel eingesetzte Alkylencarbonat Rhodium-Verbindungen in besonderer Weise stabilisieren kann. Dagegen werden im Vergleichsversuch bei Einsatz eines Alkans als Lösemittel erhebliche Rhodium-Ausfällungen sowie ein drastischer Aktivitätsverlust bei der Katalysatorrückführung beobachtet (Versuch 14 und 15). In Versuch 6 wurde die Katalysatorphase aus Versuch 5 in einer erneuten Hydroformylierung eingesetzt. Im Rahmen der experimentellen Genauigkeit bleibt der Olefin-Umsatz dabei konstant.

Die Versuche belegen, dass das erfindungsgemäße Verfahren eine deutlich höhere Chemoselektivität zu den gewünschten Aldehyden bietet und darüber hinaus eine technisch einfache Katalysatorrückführung ohne wesentliche Desaktivierung erlaubt.

## Patentansprüche

1. Verfahren zur katalytischen Hydroformylierung von olefinisch ungesättigten Verbindungen mit 3 bis 24 Kohlenstoffatomen, wobei als Katalysator ein zumindest ein Metall der 8. bis 10. Gruppe des Periodensystems der Elemente aufweisender, unmodifizierter Katalysator eingesetzt wird,
**dadurch gekennzeichnet,**
**dass** die Hydroformylierung in Gegenwart eines cyclischen Kohlensäureesters der Formel I 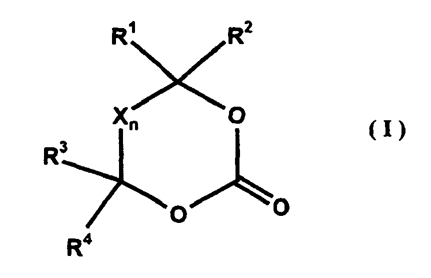 mit
R¹, R², R³, R⁴: jeweils gleich oder verschieden: H, substituierte oder unsubstituierte, aliphatische, alicyclische, aromatische, aliphatisch-alicyclische, aliphatisch-aromatische, alicyclisch-aromatische Kohlenwasserstoffreste mit 1 bis 27 C-Atomen.
n : 0-5
X: zweiwertiger substituierter oder unsubstituierter, aliphatischer, alicyclischer, aromatischer, aliphatisch-alicyclischer, aliphatischaromatischer Kohlenwasserstoffrest mit 1 bis 27 C-Atomen.
durchgeführt wird, wobei der Anteil des Kohlensäureesters zumindest 1 Gew.-% des Reaktionsgemisches beträgt.

2. Verfahren nach Anspruch 1,
**dadurch gekennzeichnet,**
**dass** R¹, R², R³, R⁴ und X gleich oder verschieden durch O, N, NH-, N-alkyl oder N-Dialkylreste, Fluor, Chlor, Brom, Jod, -OH, -OR, -CN, -C(O)alkyl oder -C(O)O-alkyl substituiert sind.

3. Verfahren nach einem der Ansprüche 1 oder 2,
**dadurch gekennzeichnet,**
**dass** die Hydroformylierung in Gegenwart von 5 bis 50 Gew.-% bezogen auf die Reaktionsmasse eines im Vergleich zum cyclischen Kohlensäureester I, unpolaren und mit dem cyclischen Kohlensäureester I nicht mischbaren Lösemittels durchgeführt wird.

4. Verfahren nach einem der Ansprüche 1 bis 3,
**dadurch gekennzeichnet,**
**dass** das Reaktionsprodukt der Hydroformylierung mit einem unpolaren und mit dem cyclischen Kohlensäureester I nicht mischbaren Lösemittel extrahiert wird.

5. Verfahren nach Anspruch 3 oder 4,
**dadurch gekennzeichnet,**
**dass** als unpolares Lösemittel substituierte oder unsubstituierte Kohlenwasserstoffe mit 10 bis 50 Kohlenstoffatomen oder Olefine mit 3 bis 24 Kohlenstoffatomen eingesetzt werden.

6. Verfahren nach einem der Ansprüche 1 bis 5,
**dadurch gekennzeichnet,**
**dass** die Hydroformylierung in Gegenwart von HRh(CO)₃ als Katalysator durchgeführt wird.

7. Verfahren nach einem der Ansprüche 1 bis 6,
**dadurch gekennzeichnet,**
**dass** der Reaktionsaustrag der Hydroformylierungsreaktion in eine überwiegend den Katalysator und den cyclischen Kohlensäureester enthaltende und eine überwiegend die Hydroformylierungsprodukte enthaltende Fraktion getrennt wird.

8. Verfahren nach einem der Ansprüche 1 bis 7,
**dadurch gekennzeichnet,**
**dass** die den Katalysator enthaltende Fraktion in die Hydroformylierungsreaktion rückgeführt wird.

9. Verfahren nach einem der Ansprüche 1 bis 8,
**dadurch gekennzeichnet,**
**dass** als cyclischer Kohlensäureester Ethylencarbonat, Propylencarbonat oder Butylencarbonat oder deren Gemische eingesetzt werden.

10. Verfahren nach einem der Ansprüche 1 bis 9,
**dadurch gekennzeichnet,**
**dass** die nicht umgesetzten olefinisch ungesättigten Verbindungen aus dem Reaktoraustrag oder von den Hydroformylierungsprodukten abgetrennt und in dieselbe Hydroformylierungsreaktion zurückgeführt oder in eine zweite Hydroformylierungsreaktion geführt werden.

11. Verfahren nach einem der Ansprüche 1 bis 10,
**dadurch gekennzeichnet,**
**dass** als olefinisch ungesättigte Verbindungen solche eingesetzt werden, die als nicht umgesetzte olefinisch ungesättigte Verbindungen aus dem Reaktoraustrag einer ersten Hydroformylierungsreaktion erhalten wurden.

12. Verfahren nach Anspruch 11
**dadurch gekennzeichnet,**
**dass** als olefinisch ungesättigte Verbindungen solche eingesetzt werden, die als nicht umgesetzte olefinisch ungesättigte Verbindungen aus dem Reaktoraustrag einer ersten Hydroformylierungsreaktion, die in Gegenwart eines Ligand-modifizierten Katalysators durchgeführt wurde, erhalten wurden.

## Claims

1. Process for the catalytic hydroformylation of olefinically unsaturated compounds having from 3 to 24 carbon atoms using an unmodified catalyst comprising at least one metal of groups 8 to 10 of the Periodic Table of the Elements, **characterized in that** the hydroformylation is carried out in the presence of a cyclic carbonic ester of the formula I 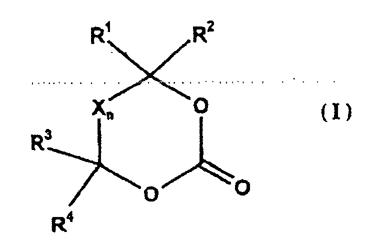 where
R¹, R², R³, R⁴ are identical or different and are each H or a substituted or unsubstituted aliphatic, alicyclic, aromatic, aliphatic-alicyclic, aliphatic-aromatic or alicyclicaromatic hydrocarbon radical having from 1 to 27 carbon atoms,
n is 0 - 5
X is a divalent substituted or unsubstituted, aliphatic, alicyclic, aromatic, aliphatic-alicyclic or aliphatic-aromatic hydrocarbon radical having from 1 to 27 carbon atoms,
with the proportion of the carbonic ester being at least 1% by weight of the reaction mixture.

2. Process according to Claim 1, **characterized in that** R¹, R², R³, R⁴ and X are substituted by identical or different substituents selected from among O, N, NH, N-alkyl and N-dialkyl radicals, fluorine, chlorine, bromine, iodine, -OH, -OR, - CN, -C(O)alkyl or -C(O)O-alkyl.

3. Process according to Claim 1 or 2, **characterized in that** the hydroformylation is carried out in the presence of from 5 to 50% by weight, based on the reaction mixture, of a solvent which is nonpolar compared to the cyclic carbonic ester I and is immiscible with the cyclic carbonic ester I.

4. Process according to any of Claims 1 to 3,
**characterized in that** the reaction product from the hydroformylation is extracted with a nonpolar solvent which is immiscible with the cyclic carbonic ester I.

5. Process according to Claim 3 or 4, **characterized in that** substituted or unsubstituted hydrocarbons having from 10 to 50 carbon atoms or olefins having from 3 to 24 carbon atoms are used as nonpolar solvent.

6. Process according to any of Claims 1 to 5,
**characterized in that** the hydroformylation is carried out in the presence of HRh(CO)₃ as catalyst.

7. Process according to any of Claims 1 to 6,
**characterized in that** the reaction product mixture from the hydroformylation reaction is separated into a fraction comprising predominantly the catalyst and the cyclic carbonic ester and a fraction comprising predominantly the hydroformylation products.

8. Process according to any of Claims 1 to 7,
**characterized in that** the fraction comprising the catalyst is recirculated to the hydroformylation reaction.

9. Process according to any of Claims 1 to 8,
**characterized in that** the cyclic carbonic ester used is ethylene carbonate, propylene carbonate or butylene carbonate or a mixture thereof.

10. Process according to any of Claims 1 to 9,
**characterized in that** the unreacted olefinically unsaturated compounds are separated off from the reactor output or from the hydroformylation products and are returned to the same hydroformylation reaction or passed to a second hydroformylation reaction.

11. Process according to any of Claims 1 to 10,
**characterized in that** the olefinically unsaturated compounds used are compounds which have been obtained as unreacted olefinically unsaturated compounds from the reactor output of a first hydroformylation reaction.

12. Process according to Claim 11, **characterized in that** the olefinically unsaturated compounds used are compounds which have been obtained as unreacted olefinically unsaturated compounds from the reactor output of a first hydroformylation reaction carried out in the presence of a ligand-modified catalyst.

## Revendications

1. Procédé d'hydroformylation catalytique de composés oléfiniquement insaturés contenant 3 à 24 atomes de carbone, un catalyseur non modifié contenant au moins un métal des groupes 8 à 10 du tableau périodique des éléments étant utilisé en tant que catalyseur,
**caractérisé en ce que**
l'hydroformylation est réalisée en présence d'un ester d'acide carbonique cyclique de formule I 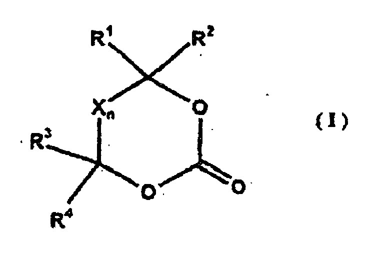 avec
R¹, R², R³, R⁴ : à chaque fois identiques ou différents : H, radicaux hydrocarbonés substitués ou non substitués, aliphatiques, alicycliques, aromatiques, aliphatiques-alicyclique, aliphatiques-aromatiques, alicycliques-aromatiques, contenant 1 à 27 atomes C
n : 0 à 5
X : radical hydrocarboné bivalent substitué ou non substitué, aliphatique, alicyclique, aromatique, aliphatique-alicyclique, aliphatique-aromatique, contenant 1 à 27 atomes C,
la proportion de l'ester d'acide carbonique représentant au moins 1 % en poids du mélange réactionnel.

2. Procédé selon la revendication 1,
**caractérisé en ce que**
R¹, R², R³, R⁴ et X sont substitués de manière identique ou différente par O, N, NH-, des radicaux N-alkyle ou N-dialkyle, fluor, chlore, brome, iode, -OH, -OR, -CN, -C(O)alkyle ou -C(O)O-alkyle.

3. Procédé selon l'une quelconque des revendications 1 ou 2,
**caractérisé en ce que**
l'hydroformylation est réalisée en présence de 5 à 50 % en poids, par rapport à la masse réactionnelle, d'un solvant non polaire en comparaison de l'ester d'acide carbonique cyclique I et non miscible avec l'ester d'acide carbonique cyclique I.

4. Procédé selon l'une quelconque des revendications 1 à 3,
**caractérisé en ce que**
le produit de réaction de l'hydroformylation est extrait avec un solvant non polaire et non miscible avec l'ester d'acide carbonique cyclique I.

5. Procédé selon la revendication 3 ou 4,
**caractérisé en ce que**
des hydrocarbures substitués ou non substitués contenant 10 à 50 atomes de carbone ou des oléfines contenant 3 à 24 atomes de carbone sont utilisés en tant que solvant non polaire.

6. Procédé selon l'une quelconque des revendications 1 à 5,
**caractérisé en ce que**
l'hydroformylation est réalisée en présence d'HRh(CO)₃ en tant que catalyseur.

7. Procédé selon l'une quelconque des revendications 1 à 6,
**caractérisé en ce que**
la sortie de la réaction d'hydroformylation est séparée en une fraction contenant principalement le catalyseur et l'ester d'acide carbonique cyclique et une fraction contenant principalement les produits de l'hydroformylation.

8. Procédé selon l'une quelconque des revendications 1 à 7,
**caractérisé en ce que**
la fraction contenant le catalyseur est recyclée dans la réaction d'hydroformylation.

9. Procédé selon l'une quelconque des revendications 1 à 8,
**caractérisé en ce que**
du carbonate d'éthylène, du carbonate de propylène ou du carbonate de butylène ou leurs mélanges sont utilisés en tant qu'ester d'acide carbonique cyclique.

10. Procédé selon l'une quelconque des revendications 1 à 9,
**caractérisé en ce que**
les composés oléfiniquement insaturés non réagis sont séparés de la sortie du réacteur ou des produits de l'hydroformylation et recyclés dans la même réaction d'hydroformylation ou dans une seconde réaction d'hydroformylation.

11. Procédé selon l'une quelconque des revendications 1 à 10,
**caractérisé en ce que**
des composés oléfiniquement insaturés qui ont été obtenus en tant que composés oléfiniquement insaturés non réagis à partir de la sortie du réacteur d'une première réaction d'hydroformylation sont utilisés en tant que composés oléfiniquement insaturés.

12. Procédé selon la revendication 11,
**caractérisé en ce que**
des composés oléfiniquement insaturés qui ont été obtenus en tant que composés oléfiniquement insaturés non réagis à partir de la sortie du réacteur d'une première réaction d'hydroformylation, réalisée en présence d'un catalyseur modifié par un ligand, sont utilisés en tant que composés oléfiniquement insaturés.
